Europäisches Patentamt

European Patent Office

Office européen des brevets

(19)

(11) Veröffentlichungsnummer: **0 123 269**

**B1**

(12) **EUROPÄISCHE PATENTSCHRIFT**

(45) Veröffentlichungstag der Patentschrift: **02.01.91**

(21) Anmeldenummer: **84104395.3**

(22) Anmeldetag: **18.04.84**

(51) Int. Cl.⁵: **A 61 M 1/06**

(54) Frauenmilchpumpe.

(30) Priorität: **25.04.83 DE 3314942**

(43) Veröffentlichungstag der Anmeldung:
**31.10.84 Patentblatt 84/44**

(45) Bekanntmachung des Hinweises auf die
Patenterteilung:
**02.01.91 Patentblatt 91/01**

(84) Benannte Vertragsstaaten:
**CH DE FR GB LI SE**

(56) Entgegenhaltungen:
**BE-A- 558 158
DE-A-2 347 034
DE-A-2 658 322
DE-C-1 067 982
GB-A-2 106 189**

(73) Patentinhaber: **MEDAP Medizinische Apparate
Alfred Horstmann Gesellschaft mit beschränkter
Haftung
Daimlerstrasse 20 Postfach 1965
D-6380 Bad Homburg (DE)**

(72) Erfinder: **Nitsche, Klaus
Im Aichert 2/1
D-7121 Erligheim (DE)**
Erfinder: **Schütt, Peter, Dr.
Lilienweg 11/2
D-7121 Löchgau (DE)**
Erfinder: **Seidel, Johann
Sindlinger Weg 2
D-6380 Bad Homburg (DE)**
Erfinder: **Stolper, Michael
Neugasse 9a
D-6236 Eschborn (DE)**

(74) Vertreter: **Utermann, Gerd, Dipl.-Ing.
Kilianstrasse 7 (Kilianspassage) Postfach 3525
D-7100 Heilbronn (DE)**

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische
Patent Einspruch einlegen. Der Einspruch ist schriftlich einzureichen und zu begründen. Er gilt erst als eingelegt,
wenn die Einspruchsgebühr entrichtet worden ist (Art. 99(1) Europäisches Patentübereinkommen).

EP 0 123 269 B1

Courier Press, Leamington Spa, England.

**Beschreibung**

Die Erfindung betrifft eine Frauenmilchpumpe mit einer in einem Pumpengehäuse zwischen einem antriebbaren Kolben und einem eine Lufteinlaß- und -auslaßbohrung aufweisenden Deckel bewegbar angeordneten Membran, welche als Trennmembran zwischen einer Stirnfläche des Pumpengehäuses und dem vom Gehäuse abnehmbaren Deckel eingespannt ist, welche zusammen mit dem Deckel den Saugraum begrenzt, und welche im Saugrhythmus bewegbar ist.

Frauenmilchpumpen dienen dazu, Muttermilch, die nicht direkt getrunken wird, abzusaugen. Dazu wird ein Brustglas aufgesetzt, welches direkt oder über Austauschmittel mit einem Milchauffangbehälter verbunden ist und zum anderen einen Anschluß für eine Saugleitung aufweist, über die ein Unterdruck im Brustglas und ggf. ein kleiner Überdruck wechselweise erzeugt werden können. Lufteintrittsöffnungen können von Hand betätigt werden. Zur Erzeugung eines geeigneten Unterdrucks in einem Rhythmus, der in etwa dem natürlichen Trinkrhythmus entspricht, sind verschiedene Saugpumpen eingesetzt worden. Die in der Praxis am meisten verwendeten Pumpen sind langsam laufende Kolbenpumpen, die über Getriebemotoren angetrieben werden. Solche Frauenmilchpumpen mit Saugkolben sind in den deutschen Patentschriften DE—C—479 623, DE—C—844 499 und DE—C—1 067 982 beschrieben. Sie sind konstruktiv aufwendig und dadurch relativ teuer, haben jedoch vor allem eine bsondere Schwierigkeit. Wenn aus irgendwelchen Gründen nicht nur Luft, sondern Teile der Muttermilch in den Einlaß der Pumpe oder gar in den Kolbenraum gesaugt werden, so müssen die Pumpen völlig zerlegt, gereinigt und desinfiziert werden. Solche Frauenmilchpumpen, werden vielfach in Kliniken eingesetzt oder auch nacheinander an verschiedene junge Mütter ausgeliehen. Jeweils ist für entsprechende Reinigung und Desinfektion zu sorgen. Da das Hygienebewußtsein stark gewachsen ist und man der Übertragung von Keimen durch solche Einrichtungen entgegenwirken möchte, besteht ein Bedürfnis nach einfache, preiswerten Pumpen, deren Teile, die möglicherweise mit der Muttermilch in Berührung kommen können, leicht zerlegt, gereinigt und desinfiziert werden können. Diesem Bedürfnis kamen die zumeist auch konstruktiv recht aufwendigen und schweren Kolbenpumpen nicht nach.

Aus der DE—A—26 58 322 ist eine Frauenmilchmpumpe mit einer Membran bekannt. Diese wird über einen Stößel von einer Schrägflächenscheibe nach Art einer Taumelscheibenpumpe mit Hilfe eines Elektrogetriebemotors angetrieben und ist für direktes Anschließen an das elektrische Versorgungsnetz vorgesehen. Die von dem Stößel angetriebene Membran ist im Inneren fest eingespannt und kann nur vom Mechaniker nach Zerlegen der Gesamtheit herausgenommen werden. Sie ist im Saugraum mit einer Druckfeder belastet. Im Innern sind Scheibenventile untergebracht. In einem Vorraum kann durch ein einstellbares Nadelventil Nebenluft eingesaugt werden. All diese Teile sind formlich kompliziert gestaltet, mit vielen Ecken und Hinterschneidungen und liegen unter einer Gehäusekappe fest mit dem Motorgehäuse eingekapselt, so daß sie von der Wöchnerin nicht zerlegt und gereinigt werden können. Wenn Muttermilch der einen Wöchnerin durch den an sich nur für Saugluft und Druckluft vorgesehenen Schlauch in das Pumpeninnere eintritt, muß damit gerechnet werden, daß sich Bakterien im Innern gut entwickeln, zumal der unmittelbar angebaute Elektromotor für Netzspeisung auch eine entsprechende Wärmeentwicklung mit sich bringen dürfte, die dem Bakterienwachstum förderlich ist. Für den praktischen Einsatz ist deshalb eine derartige Pumpe nicht geeignet, da die Bakterien aus dem Innern der Pumpe bei jedem Druckhub wiederum in die Luftleitung und damit unmittelbar in das Brustglas einer anderen Wöcherin gefördert werden können und so zur Übertragung von Infektionserregern führen. Durchmesser und Hub der Membran sind so klein, daß das Hubvolumen viel zu klein ist, um die Pumpe in einem dem natürlichen Saugrhythmus in etwa angepaßten Rhythmus von ca. 40 bis 50 Hübe je Minute zu betreiben und dann noch geeignet abzusaugen und in den Vorratsbehälter zu fördern.

Aus der DE—C—823 641 ist eine komplifiziert geformte Saug- und Massageeinrichtung für die Brust bekannt geworden, die jedoch ebenfalls recht kompliziert aufgebaut ist und deren Teile nur schwer zu reinigen und desinfizieren sein dürften. Hier ist eine weitab liegende Luftpumpe vorgesehen.

Den nächstliegenden Stand der Technik bildet die BE—A—558 158 (Choillot)=FR—A—1 145 848 die aus dieser Druckschrift bekannte Frauenmilchpumpe hat kein Gehäuse im Sinne des Anspruches 1, weil sie als offenes, handbetätigtes Gestell ausgebildet ist. Sie hat ein kegelstumpfförmiges, einen Deckel im Sinne des Patentanspruches bildendes Metallgehäuse, welches in seinem Zentrum außen eine Einhängeöse aufweist und an seinem größten Teil einen Flansch besitzt. Auf dem Flansch ist mit Hilfe eines Abdichtungs- und Feder-Halteringes und mittels mehrerer einzelner durchgeschraubter Schrauben eine Membran eingespannt. Der Feder-Haltering entspricht in seiner Funktion der Stirnfläche des Pumpengehäuses im Sinne von Anspruch 1. Im Mittelteil ist die Membran zwischen zwei kolbenartigen Scheiben zusätzlich mittels einer durchgehenden Schraube mit Mutter eingespannt ist. An den kolbenartigen Scheiben befindet sich eine Betätigungsstangen mit einer an ihrem gegenüberliegenden Ende angeordneten Einhängeöse. Von der einhängeöse zum Abdichtungs- und Feder-Haltering erstrecken sich Zugfedern. Im Bereich der oberen Kante des kegelstumpfförmigen Deckels ist ein Rohr als Lufteinlaß und Luftauslaß vorgesehen, der mit Hilfe eines Schlauches unmittelbar ohne weitere Ventile mit dem Saugstutzen eines Brustglases verbunden ist. Die Frauenmilchpumpe wird von Hand durch Auseinanderziehen der beiden Ösen betätigt, indem die Membran entgegen der Wirkung der Zugfedern nach außen gezogen wird, so daß Unterdruck im Brustglas und der angeschlossenen Milch-Auffang-Flasche entsteht. Beim Loslassen oder Vorbewegen entsteht wiederum ein Überdruck infolge des Zurückdrückens der Federn. Die Frauenmilchpumpe ist ohne geeignete Führung für den Kolben und damit ohne sichere Betätigung gestaltet. Ein geeigneter Antrieb zur Erzeugung eines zweckmäßigen vorgegebenen Rhythmus fehlt. Vor allem ist die Pumpe trotz der in der Beschreibung geäußerten Wunschvorstellung nach hygienischer Ausgestaltung nicht für eine schnelle Reinigung und Desinfektion geeignet, weil man zum Reinigen des Innenraumes die einzelnen Schrauben der Ringeinspannung mit einem Schraubenzieher öffnen muß und weil die Kolbenstange mit Hilfe eines Mutternschlüssels abgeschraubt werden müßte, um die Membran an ihrer Inneneinspannung einwandfrei reinigen zu können. Für einen Klinikbetriebe, bei dem die Frauenmilchpumpe nacheinander von mehreren Müttern benutzt wird, ist sie vor allem auch unter hygienischen Gesichtspunkten nicht geeignet. Inwieweit eine geeignete Brustmassage ermöglicht wird, ist nicht ersichtlich.

Für ein gutes Absaugen der Frauenmilch ist auch eine gewisse Brustmassage erforderlich, für die jedoch nicht so komplizierte Vorrichtungen notwendig und zweckmäßig erscheinen. Vielmehr reicht es aus, in einem geeigneten Rhythmus im Brustglas einen geeigneten Unterdruck und einen geringen Gegendruck abwechseln zu lassen, um die Massage zu erreichen. Gerade durch das Erzeugen eines gewissen Gegendrucks ist jedoch die Gefahr der Förderung von Bakterien oder Schmutzteilen aus der Pumpe in das Brustglas gegeben, so daß für einen hygienischen Betrieb neue Lösungen zu finden sind. Hier setzt die Erfindung an.

Der Erfindung liegt die Aufgabe zugrunde, eine Frauenmilchpumpe der eingangs genannten Art derart auszugestalten, daß bei verschiedenen Möglichkeiten für den Antrieb der mit der Luft des Brustglases in Verbindung kommende Raum vom Antriebsteil für die Erzeugung von wechselndem Unterdruck und leichtem Überdruck mit Sicherheit gegen Luft-, Flüssigkeits-, Bakterien- und Schmutzübertragung dicht abgetrennt und der mit der Luft des Brustglases in Verbindung kommende Teil leicht abzunehmen, zu reinigen und zu desinfizieren ist und diese Teile leicht einsehbar sind, um hohen hygienischen Anforderungen gerecht zu werden.

Erfindungsgemäß ist vorgesehen, daß der Deckel wenigstens zum größten Teil durchsichtig ist, daß der Deckel mit einem Schnellverschluß ausgestattet ist und daß die Membran als Rollmembran ausgebildet ist, die ausschließlich an ihrem äußeren Rand eingespannt ist.

Während bei den bisher bekannten Membranpumpen die Membran im Innern eingespannt oder derart fest eingespannt und eingeschraubt war, daß sie praktisch von der Benutzerin nicht herausnehmbar war und auch die sonstigen Teile und konstruktiven Gegebenheiten nicht unter hygienischen Gesichtspunkten ausgebildet und gestaltet waren, steht nunmehr die leichte Erreichung der Hygiene im Vordergrund, und zwar durch eine stark verbesserte Trennung vom technischen und medizinischen Bereich durch eine Trennmembran, die die Wöchnerin selbste auf einfache Weise freilegen, herausnehmen und mit den übrigen Teilen reinigen und desinfizieren kann. Dadurch, daß der abnehmbare Deckel durchsichtig ist, kann sie auch ohne weiteres sehen, ob unbeabsichtigt Milch in den Saugraum gelangt ist, was eine sofortige Reinigung erfordert. Da auch bei dieser Ausbildung—wie an sich bekannt—Lufteinlaß und Luftauslaß im abnehmbaren Deckel angeordnet sind, sind auch diese der guten Reinigung zugänglich und stellen keine Ansammlungsmöglichkeiten für Rückstände und Bakterien dar. Für den Antrieb der Trennmembran eignet sich besonders eine elektromechanische oder pneumatische Kraftbetätigung. Dadurch kann wie bei einer großvolumigen Kolbenpumpe gesaugt werden, indem nun auch eine Membranpumpe das zweckmäßige Absaugen ohne eine rhythmische Betätigung eines Handventiles oder eine Fingeröffnung durch die Wöchnerin oder durch das rhythmische Auseinderziehen und Zusammenschnurrenlassen der federvorgespannten Pumpe gestattet. Die leicht entnehmbare Rollmembran liegt frei auf einem nunmehr als einfacher Stützkolben ausgebildeten Kolben auf und erfüllt dadurch einerseits einwandfreie Trennbedingungen und andererseits kann sie nach leichtem Abnehmen des Deckels ohne weiteres mit entnommen, gereinigt und desinfiziert werden. Dabei ist auch wichtig, daß der Unterdruck beim Saugen nicht zu stark werden darf, sondern zwischen −0,1 und −0,32 bar bezogen auf die Atmosphäre liegen sollte, andererseits jedoch die jeweils zur Verfügung stehende Milchmenge abzusaugen und entsprechende größere Luftmengen abzusaugen sind. So muß die Pumpe ein beträchtliches Saugvolumen aufweisen. Eine geeignete, frei ausgespannte Membran selbst mit mittlerem Einspannkolben läßt das vor allem bei Hand- und Federantrieb nicht zu. Deshalb sieht die Erfindung die Trennmembran als frei aufliegende Rollmembran vor, die sich auf einen als Stützkolben ausgebildeten Kolben frei aufliegend und leicht entnehmbar abstützen kann.

Um große Volumina zu erzielen, kann gemäß einer Weiterbildung der Erfindung der Mittelbereich der Trennmembran einen Hub ausführen, der in der Größenordnung ihres Durchmessers liegt. So können die relativ großen Volumina bewegt werden, während die Pumpe nach DE—OS 26 58 322 nur einen relativ kleinen Hub als Schwingmembran mit entsprechend geringen Volumina ausführen kann, wodurch die Gesamtheit der Pumpe stark vergrößert werden müßte, so daß eine Handhabung nur schwer möglich ist. Die abgestütze Rollmembran führt zu einer handlichen Größe des Gerätes und läßt die verschiedensten Antriebe auf ihrer technischen Seite zu.

Die Rollmembran ist einerseits starken mechanischen Einflüssen ausgesetzt und muß andererseits auch bei längerem Gebrauch absolute Dichtheit gewährleisten. Das läßt sich nach einem weiteren vorteilhaften Merkmal der Erfindung dadurch realisieren, daß die Rollmembran aus einem mit Gewebe verstärkten, desinfizierbaren Kunststoff, vorzugsweise auf Silikon-Basis besteht. Die Trennmembran kann zweckmäßig am Rand einen Einspann- und Dichtwulst aufweisen. Dieser erleichtert einerseits das Einlegen und gewährleistet andererseits nicht nur gute Zentrierung der Membran für den mechanischen Ablauf des Rollvorganges, sondern außerdem absolute Dichtheit zwischen technischem Teil und medizinischem Sauglufteil.

Der wenigstens zum größten Teil durchsichtige Deckel kann auf verschiedene Weise realisiert und befestigt werden. Er wird zweckmäßig im ganzen durchsichtig gestaltet und als einstückiges Kunststoffteil aus glasklarem Kunststoff hergestellt. Lediglich seine Anschlußteile dürften in der Regel nicht durchsichtig, allenfalls transparent gestaltet werden können. Der Deckel kann mit einer getrennten Überwurfmutter, mit Bajonettverschlüssen, sonstigen Klemm- oder Halteeinrichtungen ausgestattet sein. Zweckmäßig weist er jedoch ein Überschraubgewinde und geeignete Griffrippen auf. Beides kann im Kunststoff-Spritzgußverfahren einstückig mit dem Deckel gebildet werden. So bleibt seine Durchsichtigkeit für den Saugraum erhalten. Er ist leicht und preiswert aus geeignetem Werkstoff, der ein vielfaches Reinigen und Desinfizieren zuläßt, herzustellen und kann leicht ab- und aufgeschraubt werden. Durch das Schrauben ergibt sich auch besonders gute Dichtheit.

Im Deckel wird, vorzugsweise zentral, ein Schlauchanschlußstutzen mit einstellbarer Nebenluftöffnung angeordnet. So ist nicht ein getrenntes Nebenluftventil vorzusehen, sondern dieses kann auf dem Schlauchanschlußstutzen leicht gebildet werden.

Eine vorteilhafte, einfach herzustellende, gut zu montierende und vor allem leicht für Reinigung und Desinfektion zu demontierende Ausgestaltung nach der Erfindung sieht vor, daß der Schlauchanschlußstutzen in einer Öffnung des Deckels festgeschraubt ist und ein im Durchmesser kleineres Gewinde für eine Überwurf- und Einstellmutter trägt, welche einen konischen Spalt zwischen einem Dichtungsbereich und einem Sitz des Schlauchanschlußstutzens begrenzt und wobei die Überwurf- und Einstellmutter auf einer Einstellventilfeder abgestützt ist. Eine solch einfache Lösung besteht nur aus drei Teilen, von denen das eine, nämlich der Schlauchanschlußstutzen ohnehin vorhanden ist und nur mit einer entsprechenden Nebenluftöffnung versehen zu werden braucht. Sein Eingang im Innern des Saugraumes kann so angeordnet sein, daß der ganz nach vorn vorgeschobene Kolben mit der auf ihm liegenden Membran zur Abdichtung des Saugraumes nach Art eines Plattenventiles dient, so daß bei abgeschalteter Pumpe der Saugraum gegenüber dem Eintritt von Verunreinigungen von außen auch bei sonstigen Störungen gut geschützt ist.

Der Stützkolben der Rollmembran kann auf verschiedene Weise geführt werden. Zweckmäßig wird er in einem Zylindergehäuse geführt, welches ausreichende Bewegungsmöglichkeiten für die Rollmembran vorsieht und man stützt ihn auf einer Schraubedruckfeder ab. So kehr er automatisch in die vordere Lage mit kleinstem Raum zurück. Er braucht dann nur durch einen geeigneten Antrieb zurückgezogen zu werden. Eine solche Ausbildung läßt es nun bei einfachem Aufbau zu, die Grundanordnung mit der leicht herausnehmbaren hygienischen Membran und leicht abnehmbarem Deckel und Abstützung der Membran auf einem vorzugsweise gefederten Kolben nach den jeweiligen Bedürfnissen mit den verschiedensten Antrieben auszustatten. Man kann beispielsweise geeignete Linearantriebe mit entsprechendem Hub auf magnetischer oder elektromechanischer Basis, vor allem aber auch einen Kurbeltrieb vorsehen.

Der Kolben kann aber auch als Doppelkolben mit einer zweiten, gleichartigen Rollmembran und mit einer im Gehäuse gleitend geführten Kolbenstange ausgebildet sein und die Kolbenstange kann antreibbar sein. So entfällt die Federvorbelastung und es entfällt das großflächige Gleiten in dem Zylinder, weil dieses auf die Gleitführung der Kolbenstange beschränkt ist. Diese Ausführungen erfordern einen eigenen Antrieb für jede Muttermilchpumpe mit einem geeigneten Elektromotor oder dgl.

Eine wesentlich vielseitiger anschließbare und antreibbare Anordnung sieht vor, daß der federbelastete, die als Rollmembran ausgebildete Trennmembran stützende Kolben abgedichtet in einem Luftzylinder geführt ist, welcher über ein intermittierend schaltendes Ventil an einen Unterdruckerzeuger im Rhythmus anschaltbar und von diesem trennbar und mit Atmosphäre verbindbar ist. Geeignete Unterdruckerzeuger stehen vor allem in Kliniken überall zur Verfügung oder können auch als Zusatzaggregat preiswert aus der Serienproduktion entnommen und mit der Grundeinheit der Frauenmilchpumpe zusammengebaut werden. Es ist dann möglich, ein im Prinzip gleichartig aufgebautes Gerät mit Gehäuse, Schraubdeckel, Rollmembran, Kolben, Feder und Anschlußmöglichkeiten in größerer Stückzahl herzustellen und dem jeweiligen Einsatzzweck entsprechend mit Anschluß und ggf. Steuermitteln einfacher Art für den jeweils zur Verfügung stehenden Unterdruck zu versehen. Dazu kann vor allem ein Verbindungsanschluß für eine zentrale Sauganlage eines Krankenhauses oder dgl. vorgesehen sein. So wird die eigentliche Frauenmilchpumpe im Aufbau und in der Handhabung sehr einfach und kann am Bett der jeweiligen Wöchnerin schnell an die Zentralanlage angesteckt werden.

Wenn eine solche zentrale Sauganlage nicht zur Verfügung steht, so kann nach einem weiteren vorteilhaften Vorschlag eine Gasstrahlpumpe zur Erzeugung von Unterdruck aus einem zentralen Druckluftsystem oder getrennten Drucklufterzeuger vorgesehen sein. Da bei vielen Einsatzfällen in Kliniken, sonstigen Behandlungsräumen und dgl. keine Saugluft zur Verfügung steht, jedoch Druckluft, stellt dies eine weitere günstige Antriebsart dar, die auch dadurch gut ermöglicht wird, daß die eigentliche Frauenmilchpumpe eine abgedichtet eingespannte Membran aufweist, die sich besonders gut für einen Saugantrieb eignet. Man kann jedoch auch, wenn kein Druckluftzeuger vorhanden ist oder preiswert zur Verfügung steht, einen getrennten Unterdruckerzeuger der Pumpe zuordnen und diesen ggf. konstruktiv mit ihr in einem Gehäuse vereinigen, zumal solche kleine Saugpumpen mit elektrischem Antrieb preiswert am Markt erhätlich sind, während die Frauenmilchpumpe als solche ohnehin für den Klinikbetrieb

4

zweckmäßig als Anschlußeinrichtung für die vorhandene Sauglauft- oder Drucklufteinrichtung auszustatten ist.

Das intermittierend schaltende Ventil kann ein elektronisch mit Zeittaktsteuerung betätigtes Drei-Wege-Zwei-Stellungs-Ventil sein, welches wechselweise im gewünschten Rhythmus von beispielsweise 30 bis 40 Hübe pro Minute die Verbindung zum Sauflugterzeuger und zur Atmosphäre herstellt und unterbricht.

Eine andere besonders vorteilhafte Möglichkeit für das Verbinden zur Unterdruckquelle, Unterbrechen und Öffnen zur Atmosphäre, welche ein Zusammenbauen mit dem eigentlichen Pumpenaggregat gestattet, sieht vor, daß das intermittierend schaltende Ventil ein pneumatisch selbsttätig mit dem eigenen Unterdruck steuerndes Multivibratorpneumatikventilsystem ist. So werden keine Fremdenergien und keine besonderen durch andere Energien geschalteten Zeitglieder benötigt, sondern das ganze kann durch geeignete Auslegung der Querschnitte und Bemessung der Wege sowie Gestaltung von Ventilen realisiert werden, so daß die Frauenmilchpumpe nur einen Sauganschluß aufweist, an dem ein Unterdrucksystem mit geeignetem Unterdruck und geeigneter Saugleistung von beispielsweise −0,6 bar und etwa 12 l/min anzuschließen ist.

Ein solches Multivibratorpneumatikventilsystem weist zweckmäßig zwei wechselweise schaltende Tellerventile auf, von denen das eine mittels eines über Federn abgestützten und mit einer nur in den Endlagen des Kolbens wirksamen Schleppverbindung bewegten Stößels arbeitet und das andere mit beiderseits einer Ventilraumtrennmembran wirksamen Luftkräften und einer Feder betätigt wird. Eine solche Anordnung kann unmittelbar mit Zylinder und Kolben kleinräumig zusammengebaut werden und ist herstellungstechnisch einfach und schaltungstechnisch sicher und wirksam. Dabei wird zweckmäßig im Zylinderboden des Antriebszylinderraumes eine Zentralbohrung vorgesehen, durch welche der Stößel in die Ventilkammer reicht, in welcher er einen Ventilteller trägt, der vom Ende des unter der Wirkung der Feder und bei einströmender Atmosphärenluft erfolgenden Ausfahrhubes des Kolbens bis kurz vor Beendigung des unter Sog erfolgenden Einfahrhubes des Kolbens im Abstand von einer ihm gegenüber am Grund der Ventilkammer angeordneten Dichtscheibe liegt, welche eine Zentralausnehmung aufweist, die über Saugkanäle mit dem Sauganschluß verbunden ist.

Eine vorteilhafte Weiterbildung der Ausgestaltung des Ventilsystems, die herstellungstechnisch einfach mit relativ wenigen Teilen in dem Zylinderkopf gebildet werden kann, sieht vor, daß das Federventil des Multivibratorpneumatikventilsystems eine Ringdichtung aufweist, deren freier Zentralraum über eine Atmosphärenleitung mit der Atmosphäre verbunden ist und auf welche ein ringförmig dichtendes Tellerventilverschlußelement durch die Feder aufdrückbar ist, welches an der Ventilraumtrennmembran befestigt ist, die den Steuerventilraum in einen Tellerventilraum und einen Federventilraum unterteilt, wobei der Tellerventilraum über eine Leitung mit dem Antriebszylinderraum in Verbindung steht und der Federventilraum mit dem Sauganschluß in Verbindung steht und die Feder und die Wirkflächen beiderseits der Ventilraumtrennmembran derart aufeinander abgestimmt sind, daß die Verbindung zur Atmosphäre solange unterbrochen ist, wie auf beiden Seiten der Ventilraumtrennmembran etwa gleicher Druck herrscht, während beim Verschließen des durch den Stößel betätigten Stößelventiles der Unterdruck voll im Federventilraum wirksam wird und gegen die Kraft der Feder das Tellerventilverschlußelement von der Ringdichtung abhebt und die Verbindung vom Antriebszylinderraum zur Atmosphäre öffnet. Bei der kompakten Gestaltung von Zylinder und Multivibratorpneumatikventilsystem im Kopf desselben kann zweckmäßig die Atmosphärenleitung des Multivibratorpneumatikventilsystems in einem verdickten Pumpengehäuseboden des als Zylinder ausgebildeten Pumpengehäuse zu einem Ringraum führen, der von einem Ringfilter aus Filz oder Schaumstoff umgeben und von einer Gehäuseabschlußkappe abgedeckt ist. Ferner ist es von Vorteil, wenn die Ventilraumtrennmembran des Federventils des Multivibratorpneumatikventilsystems von einem auf den Pumpengehäuseboden aufgespannten, im Durchmesser im wesentlichen gleich großen Kopfstück gehalten ist und wobei in dem Kopfstück der Sauganschluß mit einem zur Achse des den Zylinder bildenden Gehäuse versetzten Sauganschlußstutzen gebildet ist und die beiden als Tellerventile ausgebildeten Ventile zentral angeordnet sind, wobei die Feder des Federventils mit einer von außen zugänglichen Einstell- und Stützschraube versehen ist. Weitere Vorteile, Einzelheiten, Merkmale und Gesichtspunkte der Erfindung sind auch dem nachfolgenden, anhand der Zeichnungen gegebenen Beschreibungsteil zu entnehmen.

Ausführungsbeispiele der Erfindung werden nachfolgend anhand der Zeichnungen näher erläutert und beschrieben.

Es zeigen:

Fig. 1 einen Längsschnitt längs der Linie 1—1 in Fig. 3 etwa im Maßstab 1:1 durch eine Frauenmilchpumpe mit unmittelbar angebautem Multivibratorpneumatikventilsystem;

Fig. 2 einen Teilschnitt längs der Linie 2—2 in Fig. 3 durch das Multivibratorpneumatikventilsystem der Frauenmilchpumpe nach Fig. 1 in einer um 90° zu Fig. 1 gedrehten Darstellung zur Veranschaulichung der Lage der verschiedenen Kanäle;

Fig. 3 eine Draufsicht auf den Kopf der Frauenmilchpumpe, die nur den abschraubbaren Deckel und das Einstellventil zeigt;

Fig. 4 eine schematische Darstellung für die verschiedenen Einsatzmöglichkeiten der Frauenmilchpumpe mit Multivibratorpneumatikventilsystem nach den Fig. 1 bis 3;

Fig. 5 eine schematische Darstellung für den Einsatz einer Frauenmilchpumpe ohne Multivibratorpneumatikventilsystem, sondern mit Zeittaktsteuerung;

Fig. 6 die schematische Darstellung einer Frauenmilchpumpe mit Trennmembran und Kurbelantrieb des Stützkolbens, wobei die Frauenmilchpumpe im wesentlichen im Schnitt dargestellt ist;

Fig. 7 eine Schnittdarstellung einer Frauenmilchpumpe mit Doppelmembran und schematisch angedeutetem Kurbelantrieb.

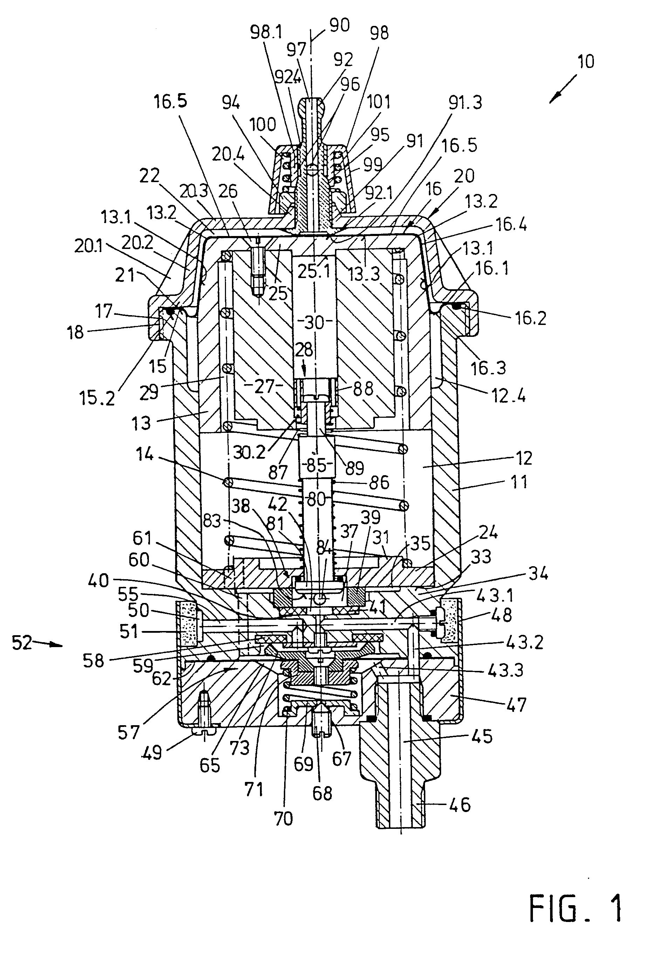

Die Frauenmilchpumpe 10 nach den Fig. 1 bis 4 hat ein Pumpengehäuse 11, welches im Inneren einen Antriebszylinderraum 12 begrenzt, in dem ein als Stützkolben ausgebildeter Kolben 13 gegen eine Druckfeder 14 bewegbar geführt ist. Auf der hier als Stirnbegrenzung des Pumpengehäuses 11 ausgebildeten Gehäusefläche 15 liegt der Rand 16.1 einer Rollmembran 16, die einen Einspann- und Dichtwulst 16.2 aufweist, der in einer Einspann- und Dichtungsnut 15.2 liegt. Der Außenbereich ist mit einem Außengewinde 17 versehen, auf dem der Innengewinderand 18 eines abnehmbaren Deckels 20 aufgeschraubt ist, so daß die Deckeldichtfläche 21 auf den Rand 16.1 der Rollmembran 16 drückt und den Einspann- und Dichtwulst 16.2 in die Einspann- und Dichtungsnut 15.2 drückt, so daß die Rollmembran 16 gasdicht, flüssigkeitsdicht und bakterien- sowie schmutzdicht eingespannt ist. Sie trennt den Saugraum 22 vom Antriebszylinderraum 12 hermetisch ab. In Fig. 1 ist die auch in der Endlage verbleibende Anfangswelle 16.3 der Rollmembran dargestellt. Der Seitenwandbereich 16.4 der Rollmembran 16 liegt an einer leicht konischen Stützfläche 13.1 des Kolbens 13 in der äußeren Stellung an und geht bei einem Abstützradius 13.2 des Kolbens 13 in den Mittelbereich 16.5 der Rollmembran über, der sich auf der ebenen Stirnfläche 13.3 des Kolbens 13 ständig abstützt. Die Rollmembran kann von der Anfangswelle 16.3 ausgehend sich in den Rollstützraum 12.4 im Inneren des Pumpengehäuses 11 hineinrollen, wenn der Kolben in Richtung auf den Zylinderboden 24 gezogen wird. Am Kolbenboden 25 ist mit Hilfe von Schrauben 26 ein Verdrängungs- und Führungsteil 27 angeschraubt, welches das Volumen des leer zu saugenden Antriebszylinderraumes soweit als möglich verkleinert und eine Führungsbohrung 30 für die Schleppverbindung 28 bildet und andererseits den Ringraum 29 für die Druckfeder 14 begrenzt.

Der Zylinderboden 24 ist von einem Einlegeteil gebildet, welches einen Federzentrierungskragen 31 aufweist und eine Zentralbohrung 32 hat. Er liegt auf einer Stützfläche 33 des Pumpengehäusebodens 34 auf, in welchem ein Ringraum 35 für die Verbindung zum Antriebszylinderraum 12 vorgesehen ist. Innerhalb dieses Ringraumes 35 ist abgesetzt die Ventilkammer 37 des stößelbetätigten Tellerventiles gebildet, welches als Stößelventil 38 bezeichnet ist. In dieser Ventilkammer 37 liegt ein Dichtungshaltering 39, der zwischen sich und dem Ventilkammerboden 40 die Dichtscheibe 41 einspannt. Diese hat eine Zentralausnehmung 42, die über Saugkanäle 43.1 und 43.2 mit dem Sauganschluß 45 in Verbindung steht. Der Sauganschluß 45 ist in einem Sauganschlußstutzen 46 gebildet, welcher in dem Kopfstück 47 des Pumpengehäuses eingeschraubt ist. Das Kopfstück 47 ist stirnseitig auf den Pumpengehäuseboden 34 aufgesetzt und trägt eine Gehäuseabschlußkappe 48, die mit Schrauben 49 befestigt ist und einen Atmosphärenringraum 50 und das in ihm liegende Ringfilter 51 abdeckt, welches aus Filz oder Schaumstoff besteht und über den zwischen der Gehäuseabschlußkappe 48 und der schrägen Pumpengehäusefläche 53 gebildeten Atmosphärenspalt 54 Atmosphärenluft ansaugt, die zur Atmosphärenleitung 55 gelangt.

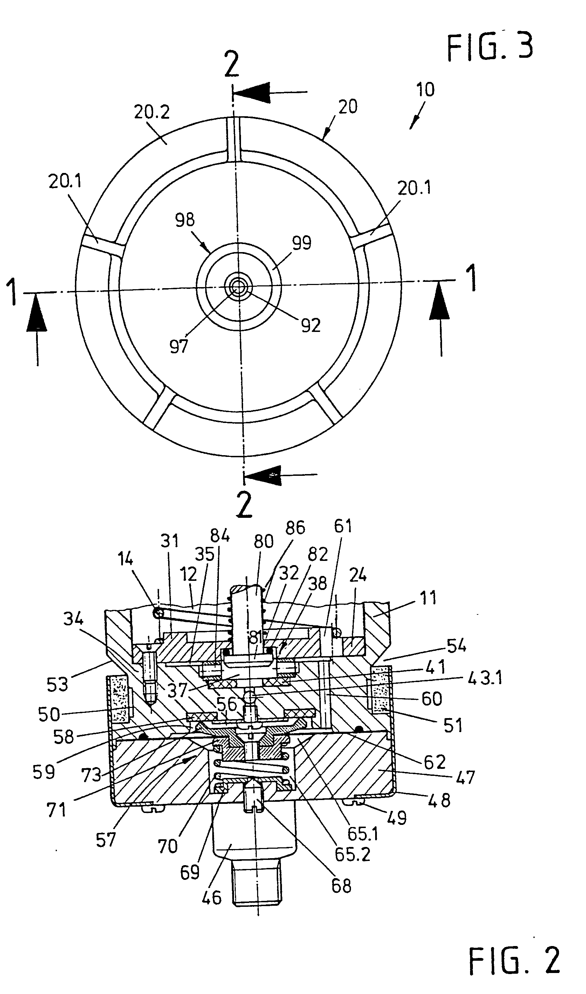

Die Atmosphärenleitung 55 führt zum freien Zentralraum 56 des als Tellerventil ausgebildeten Federventils 57. Dieses hat eine Ringdichtung 58, die von einer Haltescheibe gehalten im Tellerventilraum 59 liegt und für die Abdichtung sorgt. Der Tellerventilraum steht über eine Antriebszylinderraumleitung 60 mit dem Antriebszylinderraum 12 in Verbindung, wozu auch im Zylinderboden 24 eine Durchbrechung 61 geschaffen ist. Diese sind zur besseren Veranschaulichung der Funktion anhand der Fig. 1 in diese gestrichelt eingetragen, obwohl sie, wie aus Fig. 2 hervorgeht, um 90° zu der Anordnung nach Fig. 1 verdreht liegen.

Zwischen dem Gehäuseboden 34 und dem Kopfstück 47 ist eine flache Ventilraumtrennmembran 62 eingespannt. Diese unterteilt den Steuerventilraum des Federventils 57 in einen Tellerventilraum 59 und in einen Federventilraum 65. Der Federventilraum 65 ist in dem Kopfstück 47 ausgebildet, hat einen bis zur Ventilraumtrennmembran 62 reichenden konischen Teil 65.1 und einen zylindrischen Bereich 65.2. Dessen Boden ist mit einer Zentralgewindebohrung 67 versehen, in der eine Einstell- und Stützschraube 68 angeordnet ist. Auf dieser stützt sich der Federhalter 69 ab, der die Feder 70 trägt. Diese sitzt andererseits auf dem Tellerventilfederhalter 71, der mit Hilfe einer Schraube mit dem eigentlichen Tellerventilverschlußelement 73 durch die Ventilraumtrennmembran 62 hindurch verschraubt ist, so daß die Ventilraumtrennmembran 62 das Tellerventilverschlußelement 73 trägt und mit diesem zusammen bewegt wird. Der Federventilraum 65 ist über eine Saugbohrung 43.3 mit dem Sauganschluß 45 verbunden, jedoch durch die Ventilraumtrennmembran 62 vom Tellerventilraum 59 getrennt. Der Tellerventilraum 59 ist jedoch—wie erläutert—mit dem Antriebszylinderraum 12 verbunden.

Im Stößelventil 38 reicht durch die Zentralbohrung 32 die Stößelstange 80 bis in die Ventilkammer 37. Dort trägt sie den Ventilteller 81, der als Dämpfung auf einem O-Ring 82 gegenüber dem Zylinderboden 24 dämpfend abgestützt ist. Der Ventilteller 81 hat eine ebene Ventildichtfläche 83, die in der in den Fig. 1 und 2 dargestellten Position in einem gewissen Abstand von etwa 3 mm von der Dichtscheibe 41 liegt, so daß in dieser Stellung die Saugleitung 43 über die Zentralöffnung 42 und die Seitenbohrungen 84 in dem Dichtungshaltering 39 mit dem Ringraum 35 in Verbindung steht und dadurch in dieser Position des Ventils mit dem Antriebszylinderraum 12 verbunden ist.

Der Stößel 80 hat in seinem oberen, in der gezeichneten Stellung außerhalb des Führungs- und Verdrängungselementes 27 liegenden Bereich eine Federabstützung 85. Zwischen dieser und dem

EP 0 123 269 B1

Gehäuseboden 24 ist eine Rückholfeder 86 eingespannt, die den Stößel 80 umgibt und ihn in der dargestellten Position hält bzw. bei entsprechenden Druckverhältnissen in diese zurückzieht. Auf der Federabstützung 85 stützt sich nach oben hin eine Pufferfeder 87 ab, die andererseits auf einem Schleppanschlagelement 88 abgestützt ist, welches mit Hilfe einer Schraube 89 im Stößel 80, vorzugsweise unter Festspannung der Federabstützung 85 geringfügig verschiebbar befestigt ist.

Die Führungsbohrung 30 hat im unteren Bereich einen Anschlagabsatz 30.2 und endet oben an der Anschlagfläche 25.1 eines Zapfens des Kolbenbodens 25. Beim Zurückziehen des Kolbens 13 aus der in Fig. 1 dargestellten Position verbleiben Stößel und Schleppanschlagelement 88 solange in der dargestellten Position bis die Anschlagfläche 25.1 an das obere Ende des Schleppanschlagelementes 88 anschlägt. Dann wird unter Zusammendrückung der Pufferfeder 87 der Stößel 80 entgegen der Wirkung der Rückholfeder 86 aus der dargestellten Position mit seiner Dichtfläche 83 auf die Dichtscheibe 41 gedrückt, so daß das Stößelventil 38 verschlossen ist und die Verbindung vom Sauganschluß 45 zum Antriebszylinderraum 12 unterbunden ist und der Sog in der Zentralöffnung 42 der Dichtscheibe 41 den Ventilteller 81 und den Stößel 80 solange festhält, bis nach Ausfahren des Kolbens 13 der anschlagabsatz 30.2 an dem Schleppanschlagelement 88 zur Anlage gelangt und den Stößel 80 samt Teller 81 von der Dichtscheibe 41 abhebt, woraufhin die Stützfeder 86 den Rest des zur Verfügung stehenden Weges überwindet und den Stößel 80 mit seinem Ventilteller 81 gegen den Dämpfungsring 82 zieht, so daß gleichzeitig das Schleppanschlagelement 88 einen gewissen Abstand vom Anschlagabsatz 30.2 erhält, wie es in Fig. 1 dargestellt ist.

Der Schraubdeckel 20 besteht aus im Spritzgußverfahren hergestelltem, durchsichtigem, sterilisierbar und desinfizierbaren Kunststoff und hat, wie aus den Fig. 1 und 2 ersichtlich, am Außenumfang Griffrippen 20.1, von dene hier fünft Stück dargestellt sind. Auch andere Anzahlen sind möglich. Die Außenwand 20.2 folgt ziemlich genau der Kopfform es Kolbens 13, um einen möglichst kleinen Restraum zu bilden.

Im Zentrum des Deckelbodens 20.3, also um die Achse 90 der ganzen Frauenmilchpumpe herum, ist am Deckelboden einen Schraubensitzwulst 20.4 gebildet, der eine Öffnung 91 umgibt. In diese ist der Schlauchanschlußstutzen 92 von innen her eingesteckt. Er hat einen Anschlagkopf 92.1, mit dem er sich an den Deckelboden 20.3 von innen anlegt. Dieser ist mit einem spitzen Auslaufrand gebildet und in seinem inneren Bereich mit seinem Dichtringsitz 91.3 ausgestattet, an dem die Trennmembran 16 zwecks Abdichtung—wie in Fig. 1 dargestellt—zur Anlage kommen kann, wenn der Kolben 13 ganz ausgefahren ist.

Der Schlauchanschlußstutzen 92 ist gestuft ausgeführt. Auf seinem großen Gewinde sitzt eine Befestigungs- oder Kontermutter 94. An das große Gewinde schließt sich ein konischer Sitz 95 an. Oberhalb des konsichen Sitzes 95 sind Nebenluftbohrungen 96 vorgesehen, die von der Hauptsaugbohrung 97 abzweigen. Nach oben schließt sich das kleinere Gewinde 92.4 an. Auf diesem ist eine Überwurf- und Einstellmutter 98 aufgeschraubt, die innen einen mit konischer Dichtfläche ausgestatteten, einstückig ausgebildeten Einstellkragen 98.1 aufweist, der mit einem Innengewinde ausgestattet ist.

Nach außen ist die Überwurfmutter 98 mit einer kegelstumpfförmigen Griffaußenwand 99 abgeschlossen. Im Zwischenraum 100 liegt eine Einstellventilfeder 101, die als Druckfeder ausgebildet ist und die Überwurfmutter 98 in der jeweiligen Position fixiert.

Schlauchanschlußstutzen 92, Kontermutter 94 und Überwurf- und Einstellmutter 98 sowie Einstellventilfeder 101 bestehen zweckmäßig aus nichtrostendem Stahl oder einem anderen rostfreiem und von den in Betracht kommenden Chemikalien für die Reinigung und Desinfektion nicht angreifbaren Werkstoff. Der Deckel ist zweckmäßig einstückig im Spritzgußverfahren aus einem Polysulfon (z.B. Udel® von Union Carbide) (einem volltransparenten, spritzfähigen Kunststoff, der widerstandsfähig gegen chemische Reinigungs- und Desinfektionsmittel und Sterilisation im Autoklaven ist) hergestellt.

Pumpengehäuse 11, Kopfstück 47, Kolben 13 und Verdrängungs- und Führungsteil 27 sowie Stößel 80 mit Federabstützung 85 und Ventilteller 81 bestehen zweckmäßig aus einem Kunststoff aus der Gruppe der Polyacetate, z.B. Polyoxymethylen (POM)—wie Delrin® von DuPont, oder einem teilkristallinen thermoplastischem Polyester auf der Basis von Polybutylenterephthalat (PBT), welches auch als Polytetramethylenterephtalat (PTMT) bezeichnet wird, wie z.B. Ultradur® von BASF. Aus gleichem Werkstoff oder einem anderen mit dem Werkstoff des Verdrängungs- und Führungsteil 27 eine geeignete Gleitpaarung bildenden Werkstoff besteht das Schleppanschlagelement 88. Die Dichtscheibe 41 und die Ringdichtung 58 bestehen zweckmäßig aus einem Kautschukwerkstoff auf Silikonbasis, der nach Möglichkeit auch die Sterilisation im Autoklaven zuläßt. Tellerventilverschlußelement 73, Federhalter 69 und Tellerventilfederhalter 71 können aus Metall oder evtl. z.T. aus einen geeigneten, ebenfalls nach Möglichkeit im Autoklaven sterilisationsfähigen Kunststoff bestehen. Die Ventilraumtrennmembran 62 kann aus einem geeigneten Membranwerkstoff, beispielsweise auf Basis von künstlichem oder natürlichem Kautschuk ggf. unter Verwendung von Silikon und ggf. mit Gewebeinlage hergestellt sein. Auch sie sollte sterilisationsfähig sein.

Wichtig ist der Werkstoff der Trennmembran 16, die als Rollmembran ausgebildet ist und zweckmäßig eine Gewebeverstärkung aufweist, da sie auch bei längerem Gebrauch und weniger Achtsamkeit nicht reißen darf. Sie wird zweckmäßig aus einem hochwertigen, gegen chemische Reinigungs- und Desinfektionsmittel sowie Sterilisation im Autoklaven widerstandsfähigen Kunststoff oder Mischwerkstoff, insbesondere auf Silikonbasis hergestellt. Die übrigen Federn und die Gehäuseabschlußkappe sollten aus

7

rostfreiem Stahl bestehen, damit das ganze Gerät gut reinigungs- und stereilisationsfähig ist.

Die Arbeitsweise der Frauenmilchpumpe 10 ist folgende:

Am Sauganschlußstutzen 46 wird die Frauenmilchmpumpe 10 mit einer geeigneten Saufluftquelle, wie sie weiter unten näher erläutert werden wird, angeschlossen. Diese stellt ständig einen etwa gleichmäßigen Unterdruck von beispielsweise etwa −0,6 bar zur Verfügung und kann etwa 12 l/min absaugen. Hat sie eine größere Saugleistung, so ist zweckmäßig eine geeignete Drossel einzubauen.

Am Schlauchanschlußstutzen 92 wird ein üblicher elastischer Schlauch aufgesteckt, der zum Brustglas führt. Dieses Brustglas hat keine von Hand zu betätigende Öffnung. Die Vorrichtung befindet sich dann in der entspannten Lage, wie sie in Fig. 1 gezeigt ist. In allen Räumen herrscht Atmosphärendruck. Wird nun am Schlauchanschlußstutzen 46 die Saugluft beispielsweise durch Anstecken des Schlauches oder Betätigung eines Ventils wirksam, so gelangt sie durch das offene Stößelventil 38 in Verbindung mit dem Antriebszylinderraum 12. Die Feder 70 hält das Federventil 57 und damit die Verbindung zur Atmosphäre geschlossen. Folglich saugt die Saugluftquelle die Luft aus dem Antriebszylinderraum 12 ab und der Kolben 13 wird entgegen der Wirkung der Feder 14 zurückgezogen. Dabei gleitet das Schleppanschlagelement 88 in der Führungsbohrung 30 bis es an die Anschlagfläche 25.1 anschlägt. Solange hält die Rückholfeder 86 den Ventilteller 81 von der Dichtscheibe 41 entfernt, ggf. unterstützt von den Differenzdrucken auf den Wirkflächen. Beim weiteren Einsaugen des Kolbens 13 wird die Pufferfeder 87 zusammengedrückt und der Ventilteller 81 aus seiner Ruheposition auf die Dichtscheibe 41 gedrückt. Dadurch wird die Verbindung zum Sauganschluß 45 unterbrochen. Nun wirkt die Saugluft nur noch im Federventilraum 65 und zieht in Folge der Trennung durch die Ventilraumtrennmembran 62 durch den weiter absinkenden Unterdruck das Tellerventilverschlußelement 73 entgegen der Wirkung der Feder 70 von der Ringdichtung 58 ab, so daß Atmosphärendruck über die Antriebszylinderraumleitung 60 und die Durchbrechung 61 in den Antriebszylinderraum 12 gelangen kann. Durch entsprechende Dimensionierung der hier zur Verfügung stehenden Strömungsquerschnitte strömt nun allmählich Atmosphärenluft in den Antriebszylinderraum und gestattet es dem Kolben 12 unter der Wirkung der Feder 14 wieder auszufahren, bis der Anschlagabsatz 30.2 gegen das Schleppanschlagelement 88 anschlägt und den Stößel 80 anhebt, wodurch der Ventilteller 81 von der Dichtscheibe 41 abhebt und nunmehr die Verbindung zum Sauganschluß 45 öffnet, so daß der Sog wieder im Antriebszylinderraum 12 wirksam wird und ein neuer Zyklus beginnt. In dieser Stellung herrscht etwa gleicher Druck auf beiden Seiten der Ventilraumtrennmembran 62, in dem Federventilraum 65 und dem Tellerventilraum 59, so daß die Kraft der Feder 70 wirksam wird und das Tellerventilverschlußelement 73 auf seine Ringdichtung 58 drückt und damit die Verbindung zur Atmosphäre verschließt. Durch diesen, von den Druck- und Strömungsverhältnissen sowie der Federkraft abhängigen Schaltvorgang wird auch ein weiches Umschalten gewährleistet. Die soeben beschriebene Anordnung ist eine Multivibratoranordnung, die entsprechend der Dimensionierung und den anstehenden Druck auf etwa 40 bis 50 Hübe je Minute auszulegen ist, was etwa dem natürlichen Saugrhythmus entspricht. Die Multivibratoranordnung ist hier als Multivibratorpneumatikventilsystem bezeichnet, weil sie aus zwei Ventilen und weiteren Hilfseinrichtungen besteht. Die Gesamtheit dieser Teile ist mit 52 bezeichnet. Mit Hilfe der Einstellschraube 68 wird die Vorspannung der Feder 70 so eingestellt, daß im Saugraum 22 kein größerer Unterdruck als etwa −0,35 bar gegenüber Atmosphäre auftreten kann, damit kein zu großer Sog an der Brust entstehen kann. So kann durch Einstellung eines ohnehin vorhandenen Multivibratorventils gleichzeitig ein Druckbegrenzungsventil für den zulässigen stärksten Unterdruck geschaffen werden und ein weiteres Unterdruckventil entfällt und die Gefahr einer schmerzhaften und schädlichen Überlastung des Brustgewebes ist einfach vermieden. Eine solche Multivibratorventilanordnung ist verschleißfrei und sehr geräuscharm. Rastfedern, Schaltnockeln, Kipphebel usw., die dem Verschleiß unterliegen und Geräusche erzeugen würden, entfallen. Sie ist auch einfach herstellbar und sehr wirksam, wenn sie auch in der Beschreibung aufwendig erscheint. Ein großer Vorzug ist, daß sie ohne Fremdenergie allein mit dem ohnehin erforderlichen Unterdruck arbeitet.

Die eigentliche Saugwirkung der Pumpe kommt jedoch dadurch zustande, daß der Kolben 13 sich rhythmisch mit einer Hubzahl von beispielsweise 40 bis 50 Hübe je Minute bewegt, wobei eine solche Bewegung auch mechanisch erzeugt sein kann. Der Hub liegt in der Größenanordnung des Durchmessers des Kolbens 13 und der Trennmembran 16 bzw. ihrer freien Einspannfläche. Beim Einziehen des Kolbens 13 rollt nun die Trennmembran 16 in den Rollstützraum 12.4 ein. Der Saugraum 22 vergrößert sich und bewirkt dadurch ein Absaugen der Luft aus dem Schlauchanschlußstutzen 92 mit seiner Hauptsaugbohrung 97 und damit aus dem Brustglas, so daß die übliche und bekannte Saugwirkung an der Brust auftritt. Die gewünschte Saugwirkung kann nun bis zur durch das Federventil 57 vorgegebenen Maximalsaugwirkung noch durch Einstellung der Überwurf- und Einstellmutter 98 durch leichtes Auf- und Abschrauben einreguliert werden. Dabei gelangt zwischen dem konischen Sitz 95 und dem Einstellkragen 98.1 Atmosphärenluft gedrosselt durch die Nebenluftbohrungen 96 in den Saugraum 22. So ist eine Feineinstellung der aus dem Brustglas abzusaugenden Luftmenge und damit Bestimmung des Unterdrucks möglich. Desweiteren ist gewährleistet, daß am Ende des Saughubes, wenn nämlich der Kolben 13 am Zylinderboden 24 angekommen ist, eine gewisse ausreichende Luftmenge durch die Nebenluftbohrungen 96 eingesaugt ist, um am Ende des Vorwärtshubes, also der Verkleinerung des Saugraumes 22 einen gewissen kleinen Überdruck in der Hauptsaugbohrung 97 zu erzeugen, der sich als geringer Überdruck im Brustglas auswirkt und zu einer erwünschten leichten Massage und Entspannung für den einwandfreien Milchfluß führt und die Milch in einen Vorratsbehälter oder—raum fließen läßt.

Geringe Schwankungen in der Hubfrequenz, die sich durch unterschiedliche Einstellungen des Soges an der Überwurf- und Einstellmutter 98 ergeben, bringen keine Nachteile beim Abpumpen vom Muttermilch. Die Überwurf- und Einstellmutter 98 ist auch bei der höchstmöglichen Sogeinstellung nicht bis zum Anschlag auf dem konischen Sitz 95 zugedreht. Dadurch ist die Mindestmenge an Nebenluft, die für den Gegendruck benötigt wird, gewährleistet.

Während des Hin- und Hergehens des Kolbens 13 folgt die Trennmembran 16 stets dem Kolben und rollt auf den für sie bestimmten Flächen in üblicher Weise ohne die Gefahr einer Beschädigung ab. Sie trennt somit den Saugraum, also den hygienischen Anforderungen besonders unterliegenden Raum völlig vom technischen Teil der Frauenmilchpumpe ab. Weder Luft, sonstige Gase, noch Flüssigkeit noch Bakterien oder dgl. können von einer zu anderen Seite wechseln. Damit sind im Saugraum 22 höchste hygienische Bedingungen gewährleistet. Sollte durch den Saugschlauchanschlußstutzen durch Unachtsamkeit oder aus sonstigen Gründen Milch oder sonstige Verunreinigungen in den Saugraum 2 gelangen, so sind diese durch den durchsichtigen Deckel 20 gut zu erkennen. In einem solchen Fall und beim Wechsel der Frauenmilchmpumpe 10 von einer zur anderen Mutter wird der Deckel 20 abgeschraubt und die Trennmembran 16 entnommen. Beides kann gut ausgewaschen, desinfiziert und sterilisiert werden. Es ist dann auch leicht durch Einlegen und Zusammenschrauben wieder in einen einwandfreien Funktionszustand zu versetzen. Die Bedienung ist einfach und übersichtlich.

Im Falle einer defekt gewordenen Trennmembran 16—zum Beispiel durch einen Riß—tritt die Milchpumpe außer Funktion. Auf diese Weise werden schädliche Auswirkungen im Fehlerfalle (Keimverschleppung, Brustgewebeüberlastung) vermieden. Dabei tritt der Unterdruck im Antriebszylinderraum 12 über die undichte Stelle der Trennmembran 16 direkt in den Saugraum 22. Der Kolben 13 bleibt in ausgefahrener Stellung stehen und die Druckfeder 14 drückt die Trennmembran 16 über den Kolben 13 fest genug gegen den Dichtringsitz 91.3. An dem Schlauchanschlußstutzen 92 ist dann keine Saugwirkung mehr vorhanden.

Die grundsätzliche Funktion und Wirkungsweise wurde vorstehend anhand der Figuren 1 bis 3 in Einzelheiten erläutert.

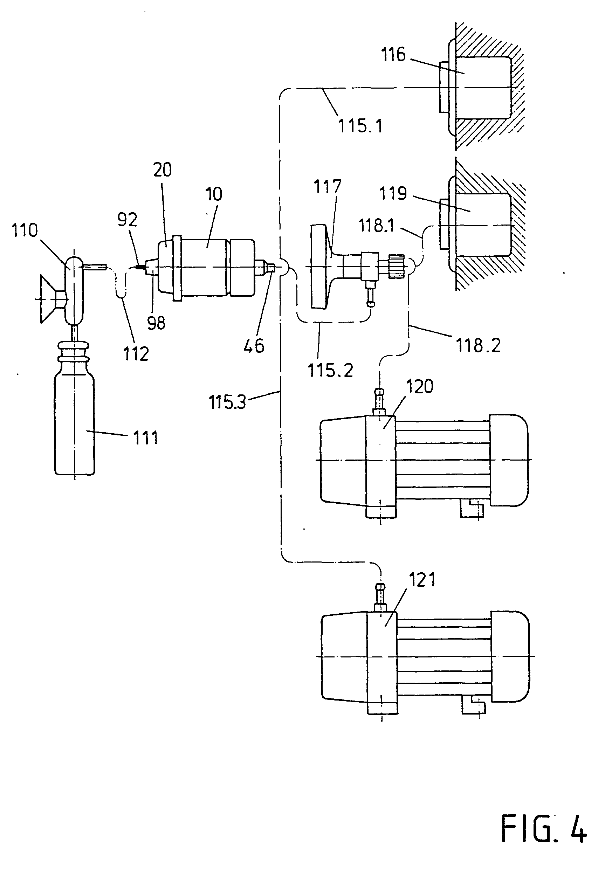

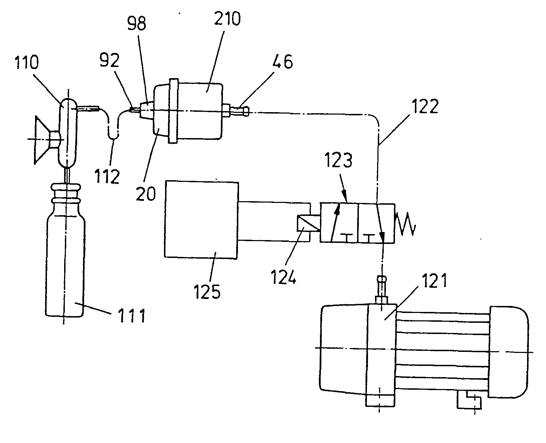

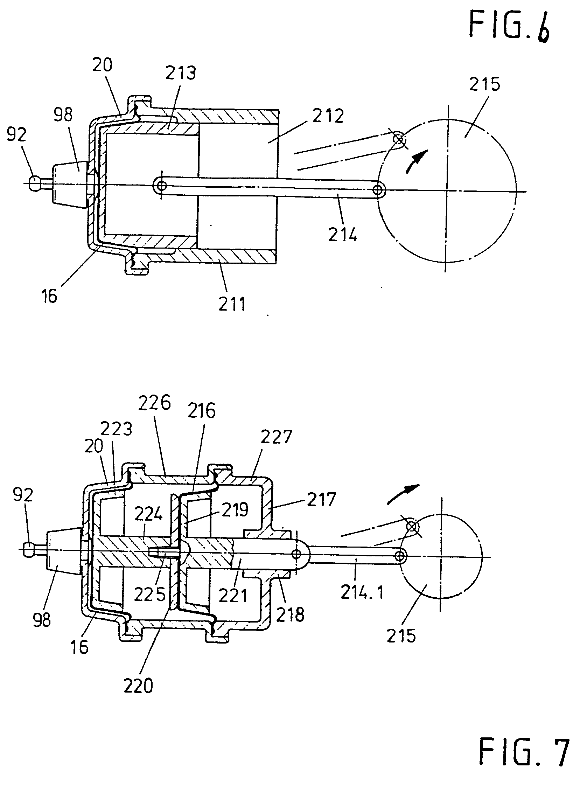

Die Fig. 4 zeigt die verschiedenen Anschlußmöglichkeiten für eine Frauenmilchpumpe 10 mit Multivibratorpneumatikventilsystem 52. Das Brustglas 110 mit der Milchflasche 111 wird über einen strichpunktiert angedeuteten Schlauch 112 mit dem Saugschlauchanschlußstutzen 92 verbunden.

An den Sauganschlußstutzen 46 können, wie die Linien 115.1, 115.2 und 115.3 veranschaulichen, verschiedene Saugquellen angeschlossen werden und zwar gemäß Linie 115.1 die Steckdose 116 einer zentralen Sauganlage eines Krankenhauses. Wenn keine zentrale Sauganlage zur Verfügung steht, so kann eine Druckluftsaugstrahlpumpe 117 eingesetzt werden, die über die Saugleitung 115.2 mit dem Sauganschlußstutzen 46 verbunden wird. Die Druckluftsaugstrahlpumpe kann nun wahlweise über die Leitung 118.1 mit der Steckdose 119 einer zentralen Druckluftversorgung verbunden und aus dieser gespeist werden. Wenn eine solche nicht vorhanden ist, kann über eine Leitung 118.2 eine Verbindung mit einem Drucklufterzeuger 120 hergestellt werden, der die Druckluftsaugstrahlpumpe 117 speist. Als weitere Alternative ist eine Verbindungsleitung 115.3 zu einem von einem Motor einzelnen angetriebenen Saugaggregat 121 schematisch dargestellte. Hier sind die vier hauptsächlichsten Speisemöglichkeiten vorgestellte, wobei die beiden unten ersichtlichen Möglichkeiten vor allem die Verbindung mit einem getrennten Saugaggregat auch zu einem einheitlichen handlichen Gerät vereinigt werden können. Solche Saugaggregate sind, wenn sie nicht ohnehin am Benutzungsort für andere Zwecke vorhanden sind, auch als sehr kleine, motorgetriebene Saugaggregate am Markt preiswert erhältlich und können mit de Frauenmilchpumpe 10 nach den Fig. 1 bis 4 in einem kleinen Gehäuse zusammengebaut werden. Das hat vor allem den Vorteil, daß das ohnehin für den Klinikbetrieb zur Verfügung zu stellende Gerät mit Multivibratorpneumatikventilsystem in größerer Stückzahl produziert und auch für den Hausgebrauch verwendet werden kann, ohne daß man eine Sonderkonstruktion schaffen muß.

Die Fig. 5 zeigt nun die Möglichkeit einer mit Saugluft betriebenen Sonderkonstruktion. Gleiche Teile sind mit gleichen Bezugszeichen versehen. Hier ist die Frauenmilchpumpe 210, wie aus dem Vergleich der Figuren ersichtlich, kürzer gezeichnet, was andeuten soll, daß ihr das Multivibratorpneumatikventilsystem fehlt. Sie hat einen abgeschlossenen Zylinderraum mit einem Anschlußstutzen 46. Dieser ist über eine Leitung 122 mit einem Zwei-Stellungsdrei-Wege-Ventil 123 verbunden. Der Elektromagnet 124 dieses Ventils ist mit einer elektronischen Taktsteuerung 125 elektrisch verbunden. Die Taktsteuerung führt zu einer Umschaltung im gewünschten Rhythmus von etwa 40 bis 50 Hüben pro Minute und kann auch mit Hubzahleinstellung ausgestattet sein. Die Leitung 122 wird so wechselweise mit dem Saugaggregat 121 bzw. der Atmosphäre verbunden. Saugluft und Feder 14 sorgen dann wechselweise für den Hub. Auch diese Frauenmilchpumpe stellt außer dem Sog auch den kleinen Gegendruck zur Verfügung.

Die Fig. 6 und 7 veranschaulichen, daß auch Kolbenpumpen mit der vorteilhaften Ausgestaltung einer den medizinischen Teil und den technischen Teil trennenden Trennmembran 16 und durchsichtigem Deckel 20 ausgestattet werden können. Gleiche Teile sind mit gleichen Bezugszeichen versehen. Das Gehäuse 211 ist in seiner unteren Stirnseite 212 offen. Der Kolben 213 ist über eine Kurbelstange 214 mit einer umlaufenden Kurbelscheibe 215 verbunden, die den üblichen Kurbelantrieb bewirkt und entsprechenden langsam angetrieben wird.

Die Fig. 7 zeigt eine gleichartig angetriebene Kurbelscheibe 215 mit einer kürzeren Pleuelstange 214.1.

Diese wirkt auf eine Kolbenstange 221, die in einem Gehäuseboden 217 in einem Schiebelager 218 verschiebbar gelagert ist. Sie ist einstückig mit einem Stützkolben 219 gebildet. Dieser trägt eine Stützscheibe 220 zum Halten der zweiten Rollmembran 216. Ein weiterer Stützkolben 223 ist mit seiner Kolbenstange 224 auf einen Stift 225 des Kolbens 219 aufgesteckt und trägt die eigentliche Trennmembran 16 in der üblichen, zuvor beschriebenen Anordnung. Hier ist jedoch ein Gehäusezwischenteil 226 vorgesehen, welches mit Innen- und Außengewinde zum Aufschrauben auf das Bodengehäuse 227 und zum Tragen des Deckels 20 ausgebildet ist. Wie ersichtlich, sind zur Führung zwei Rollmembranen vorgesehen und die Kolben habe reine Stützfunktionen für die Rollmembranen, jedoch keine Führungsfunktion an Gehäuseflächen. Eine solchen Konstruktion mit zwei Rollmembranen bringt eine noch bessere Trennung und Sicherheit und gewährleistet vor allem eine leicht gängige Führung. Auch weitere Antriebssysteme ähnlicher Art sind möglich.

Bezugszeichenliste:

| | |
|---|---|
| 10 | Frauenmilchpumpe |
| 11 | Pumpengehäuse |
| 12 | Antriebszylinderraum |
| 12.4 | Rollstützraum |
| 13 | Kolben |
| 13.1 | Konische Stützfläche |
| 13.2 | Abstützradius |
| 13.3 | ebene Stirnfläche |
| 13.4 | |
| 13.5 | |
| 14 | Druckfeder |
| 15 | Gehäusefläche |
| 15.2 | Einspann- und Dichtungsnut |
| 16 | Trennmembran |
| 16.1 | Rand |
| 16.2 | Einspann- und Dichtwulst |
| 16.3 | Anfangswelle |
| 16.4 | Seitenwandbereich |
| 16.5 | Mittelbereich |
| 17 | Außengewinde |
| 18 | Innengewinderand |
| 20 | Deckel |
| 20.1 | Griffrippe |
| 20.2 | Außenwand |
| 20.3 | Deckelboden |
| 20.4 | Schraubensitzwulst |
| 21 | Deckeldichtfläche |
| 22 | Saugraum |
| 24 | Zylinderboden |
| 25 | Kolbenboden |
| 25.1 | Anschlagfläche |
| 26 | Schraube |
| 27 | Verdrängungs- und Führungsteil |
| 28 | Schleppverbindung |
| 29 | Ringraum |
| 30 | Führungsbohrung |
| 30.2 | Anschlagabsatz |
| 31 | Federzentrierungkragen |
| 32 | Zentralbohrung |
| 33 | Stützfläche |
| 34 | Pumpengehäuseboden |
| 35 | Ringraum |
| 37 | Ventilkammer |
| 38 | Stößelventil |
| 39 | Dichtungshaltering |
| 40 | Ventilkammerboden |
| 41 | Dichtscheibe |
| 42 | Zentralausnehmung |
| 43 | Saugleitung |
| 43.1 | Saugkanal |
| 43.2 | Saugkanal |
| 43.3 | Saugbohrung |

| | |
|---|---|
| 45 | Sauganschluß |
| 46 | Sauganschlußstutzen |
| 47 | Kopfstück |
| 48 | Gehäuseabschlußkappe |
| 49 | Schraube |
| 50 | Atmosphärenringraum |
| 51 | Ringfilter |
| 52 | Multivibratorpneumatikventilsystem |
| 53 | Pumpengehäusefläche |
| 54 | Atmosphärenspalt |
| 55 | Atmosphärenleitung |
| 56 | Zentralraum |
| 57 | Federventil |
| 58 | Ringdichtung |
| 59 | Tellerventilraum |
| 60 | Antriebszylinderraumleitung |
| 61 | Durchbrechung |
| 62 | Ventilraumtrennmembran |
| 65 | Federventilraum |
| 65.1 | Konischer Teil |
| 65.2 | zylindrischer Bereich |
| 67 | Zentralgewindebohrung |
| 68 | Einstell- und Stützschraube |
| 69 | Federhalter |
| 70 | Feder |
| 71 | Tellerventilfederhalter |
| 73 | Tellerventilverschlußelement |
| 80 | Stößelstange/Stößel |
| 81 | Ventilteller |
| 82 | Dämpfungs-O-Ring |
| 83 | Ventildichtfläche |
| 84 | Seitenbohrung |
| 85 | Federabstützung |
| 86 | Rückholfeder |
| 87 | Pufferfeder |
| 88 | Schleppanschlagelement |
| 89 | Schraube |
| 90 | Achse |
| 91 | Öffnung |
| 91.3 | Dichtringsitz |
| 92 | Schlauchanschlußstutzen |
| 92.1 | Anschlagkopf |
| 92.4 | kleineres Gewinde |
| 94 | Kontermutter |
| 95 | Konischer Sitz |
| 96 | Nebenluftbohrung |
| 97 | Hauptsaugbohrung |
| 98 | Überwurf- und Einstellmutter |
| 98.1 | Einstellkragen |
| 99 | Griffaußenwand |
| 100 | Zwischenraum |
| 101 | Einstellventilfeder |
| 110 | Brustglas |
| 111 | Milchflasche |
| 112 | Schlauch |
| 115.1 | Linie |
| 115.2 | Linie/Saugleitung |
| 115.3 | Linie/Verbindungsleitung |
| 116 | Steckdose |
| 117 | Druckluftsaugstrahlpumpe |
| 118.1 | Leitung |
| 118.2 | Leitung |
| 119 | Steckdose |
| 120 | Drucklufterzeuger |
| 121 | Saugaggregat |

| 122 | Leitung |
|---|---|
| 123 | Zwei-Stellungs-Drei-Wege-Ventil |
| 124 | Elektromagnet |
| 125 | Taktsteuerung |
| 210 | Frauenmilchpumpe |
| 211 | Gehäuse |
| 212 | untere Stirnseite |
| 213 | Kolben |
| 214 | Kurbelstange |
| 214.1 | Pleuelstange |
| 215 | Kurbelscheibe |
| 216 | Rollmembran |
| 217 | Gehäuseboden |
| 218 | Schiebelager |
| 219 | Stützkolben |
| 220 | Stützscheibe |
| 221 | Kolbenstange |
| 223 | Stützkolben |
| 224 | Kolbenstange |
| 225 | Stift |
| 226 | Gehäusezwischenteil |
| 227 | Bodengehäuse. |

## Patentansprüche

1. Frauenmilchmpumpe (10) mit einer in einem Pumpengehäuse (11) zwischen einem antreibbaren Kolben (13) und einem eine Lufteinlaß- und -auslaßbohrung (97) aufweisenden Deckel (20) bewegbar angeordneten Membran, welche als Trennmembran zwischen einer Stirnfläche (15) des Pumpengehäuses und dem vom Gehäuse (11) abnehmbaren Deckel (20) eingespannt ist, welche zusammen mit dem Deckel (20) den Saugraum (22) begrenzt, und welche im Saugrhythmus bewegbar ist,
wobei der Deckel (20) wenigstens zum größten Teil durchsichtig ausgebildet und
mit einem Schnellverschluß (17, 18) ausgestattet ist, und wobei
die Membran als Rollmembran ausgebildet ist, die ausschließlich an ihrem äußeren Rand (16.1) eingespannt ist.

2. Frauenmilchpumpe nach Anspruch 1, dadurch gekennzeichnet, daß die Trennmembran (16) einen Hub ausführt, der in der Größenordnung ihres Durchmessers liegt.

3. Frauenmilchpumpe nach Anspruch 1 oder 2, dadurch gekennzeichnet, daß die Rollmembran (Trennmembran 16) aus einem mit Gewebe verstärkten, desinfizierbaren Kunststoff, vorzugsweise auf Silikon-Basis besteht.

4. Frauenmilchpumpe nach wenigstens einem der vorstehenden Ansprüche, dadurch gekennzeichnet, daß die Trennmembran (16) am Rand einen Einspann- und Dichtwulst (16.2) aufweist.

5. Frauenmilchmpumpe nach wenigstens einem der vorstehenden Ansprüche, dadurch gekennzeichnet, daß der durchsichtige Deckel (20) als einstückiges Kunststoffteil aus glasklarem Kunststoff besteht.

6. Frauenmilchmpumpe nach wenigstens einem der vorstehenden Ansprüche, dadurch gekennzeichnet, daß dem Deckel (20) ein Überschraubgewinde (Innengewinderand 18) und Griffrippen (20.1) zugeordnet sind.

7. Frauenmilchpumpe nach wenigstens einem der vorstehenden Ansprüche, dadurch gekennzeichnet, daß im Deckel (20), vorzugsweise zentral, ein Schlauchanschlußstutzen (92) mit einstellbarer Nebenluftöffnung (96) angeordnet ist.

8. Frauenmilchmpumpe nach wenigstens einem der vorstehenden Ansprüche, dadurch gekennzeichnet, daß der Schlauchanschlußstutzen (92) in einer Öffnung (91) des Deckels (20) festgeschraubt ist und ein im Durchmesser kleineres Gewinde (92.4) für eine Überwurf- und Einstellmutter (98) trägt, welche einen konischen Spalt zwischen einem Dichtungsbereich (Einstellkragen 98.1) und einem Sitz (95) des Schlauchanschlußstutzens (92) begrenzt und wobei die Überwurf- und Einstellmutter (98) auf einer Einstellventilfeder (101) abgestützt ist.

9. Frauenmilchmpumpe nach wenigstens einem der vorstehenden Ansprüche, dadurch gekennzeichnet, daß der Kolben (13, 213) der als Rollmembran ausgebildeten Trennmembran (16) in einem Zylindergehäuse (11, 211) geführt ist.

10. Frauenmilchpumpe nach wenigstens einem der vorstehenden Ansprüche, dadurch gekennzeichnet, daß der Kolben (13) auf einer Druckfeder (14) abgestützt ist.

11. Frauenmilchpumpe nach wenigstens einem der Ansprüche 1 bis 9, dadurch gekennzeichnet, daß der Kolben (13, 213) durch einen Kurbelbetrieb (214, 215) angetrieben ist.

12. Frauenmilchpumpe nach wenigstens einem der Ansprüch 1 bis 8, dadurch gekennzeichnet, daß der Kolben als Doppelkolben (223, 219) mit einer zweiten, gleichartigen Rollmembran (216) und mit einer im Gehäuse gleitend geführten Kolbenstange (221) ausgebildet ist und die Kolbenstange (221) antreibbar ist.

13. Frauenmilchpumpe nach wenigstens einem der Ansprüche 1 bis 10, dadurch gekennzeichnet, daß der federbelastete, die als Rollmembran ausgebildete Trennmembran (16) stützende Kolben (13) abgedichtet in einem Luftzylinder geführt ist, welcher über ein intermittierend schaltendes Ventil (52, 123) an einen Unterdruckerzeuger im Rhythmus anschaltbar und von diesem trennbar und mit Atmosphäre verbindbar ist.

14. Frauenmilchmpumpe nach Anspruch 13, dadurch gekennzeichnet, daß ein Verbindungsanschluß (46, 115.1, 116) für eine zentrale Sauganlage eines Krankenhauses oder dgl. vorgesehen ist.

15. Frauenmilchpumpe nach Anspruch 13, dadurch gekennzeichnet, daß eine Gasstrahlpumpe (117) zur Erzeugung von Unterdruck aus einem zentralen Druckluftsystem (118.1, 119) oder getrennten Drucklufterzeuger (118.2, 120) vorgesehen ist.

16. Frauenmilchpumpe nach Anspruch 13, dadurch gekennzeichnet, daß ein getrennter Unterdruckerzeuger (115.3, 121) der Frauenmilchpumpe (10) zugeordnet ist.

17. Frauenmilchpumpe (210) nach Anspruch 13, dadurch gekennzeichnet, daß das intermittierend schaltende Ventil ein elektronisch mit Zeittaktsteuerung (125) betätigtes Drei-Wege-Zwei-Stellungs-Ventil (123, 124) ist.

18. Frauenmilchpumpe (10) nach wenigstens einem der Ansprüche 1 bis 10 und 13 bis 16, dadurch gekennzeichnet, daß das intermittierend schaltende Ventil ein pneumatisch selbsttätig mit dem eigenen Unterdruck steuerndes Multivibratorpneumatikventilsystem (52) ist.

19. Frauenmilchpumpe nach Anspruch 18, dadurch gekennzeichnet, daß das Multivibratorpneumatikventilsystem (52) zwei Wechselweise schaltende Tellerventile (38, 57) aufweist, von denen das eine mittels eines über Federn (86, 87) abgestützten und mit einer nur in den Endlagen des Kolbens (13) wirksamen Schleppverbindung (28) bewegten Stößels (80) arbeitet und das andere mit beiderseits einer Ventilraumtrennmembran (62) wirksamen Luftkräften und einer Feder (70) betätigt wird.

20. Frauenmilchpumpe nach Anspruch 19, dadurch gekennzeichnet, daß im Zylinderboden (24) des Antriebszylinderraumes (12) eine Zentralbohrung (32) vorgesehen ist, durch welche der Stößel (80) in die Ventilkammer (37) reicht, in welcher er einen Ventilteller (81) trägt, der vom Ende des unter der Wirkung der Feder (14) und bei einströmender Atmopshärenluft erfolgenden Ausfahrhubes des Kolbens (13) bis kurz vor Beendigung des unter Sog erfolgenden Einfahrhubes des Kolbens (13) im Abstand von einer ihm gegenüber am Grund der Ventilkammer (37) angeordneten Dichtscheibe (41) liegt, welche eine Zentralausnehmung (42) aufweist, die über Saugkanäle (43.1, 43.2) mit dem Sauganschluß (45) verbunden ist.

21. Frauenmilchpumpe nach Anspruch 19 oder 20, dadurch gekennzeichnet, daß das Federventil (57) des Multivibratorpneumatikventilsystems (52) eine Ringdichtung (58) aufweist, deren freier Zentralraum (56) über eine Atmosphärenleitung (55) mit der Atmosphäre verbunden ist und auf welche ein ringförmig dichtendes Tellerventilverschlußelement (73) durch die Feder (70) aufdrückbar ist, welches an der Ventilraumtrennmembran (62) befestigt ist, die den Steuerventilraum in einen Tellerventilraum (59) und einen Federventilraum (65) unterteilt, wobei der Tellerventilraum (59) über eine Leitung (60, 61) mit dem Antriebszylinderraum (12) in Verbindung steht und der Federventilraum (65) mit dem Sauganschluß (45) in Verbindung steht und die Feder (70) und die Wirkflächen beiderseits der Ventilraumtrennmembran (62) derart aufeinander abgestimmt sind, daß die Verbindung zur Atmosphäre solange unterbrochen ist, wie auf beiden Seiten der Ventilraumtrennmembran etwa gleicher Druck herrscht, während beim Verschließen des durch den Stößel (80) betätigten Stößelventiles (38) der Unterdruck voll im Federventilraum (65) wirksam wird und gegen die Kraft der Feder (70) das Tellerventilverschlußelement (73) von der Ringdichtung (58) abhebt und die Verbindung vom Antriebszylinderraum (12) zur Atmosphäre (55, 50, 51, 54) öffnet.

22. Frauenmilchpumpe nach wenigstens einem der Ansprüche 19 bis 21, dadurch gekennzeichnet, daß die Atmosphärenleitung (55) des Multivibratorpneumatikventilsystems (52) in einem verdickten Pumpengehäuseboden (34) des als Zylinder ausgebildeten Pumpengehäuses (11) zu einem Ringraum (50) führt, der von einem Ringfilter (51) aus Filz oder Schaumstoff umgeben und von einer Gehäuseabschlußkappe (48) abgedeckt ist.

23. Frauenmilchpumpe nach wengistens einem der vorstehenden Ansprüche, dadurch gekennzeichnet, daß die Ventilraumtrennmembran (62) des Federventils (57) des Multivibratorpneumatikventilsystems (52) von einem auf den Pumpengehäuseboden (34) aufgespannten, im Durchmesser im wesentlichen gleich großen Kopfstück (47) gehalten ist, wobei in dem Kopfstück (47) der Sauganschluß (45) mit einem zur Achse (90) des den Zylinder bildenden Gehäuses (11) versetzten Sauganschlußstutzen (46) gebildet ist, die beiden als Tellerventile ausgebildeten Ventile (Stößelventil 38 und Federventil 57) zentral angeordnet sind, und die Feder (70) des Federventils (57) mit einer von außen zugänglichen Einstell- und Stützschraube (68) versehen ist.

**Revendications**

1. Pompe à lait maternel (10) avec une membrane disposée de façon déplaçable dans un corps de pompe (11) entre un piston (13) actionnable et un couvercle (20) présentant un alésage d'entrée et de sortie d'air (97), laquelle membrane est serrée sous forme d'une membrane de séparation entre une surface frontale (15) du corps de pompe et le couvercle (20) séparable du corps (11), limite la chambre d'aspiration (22) en même temps que le couvercle, et est déplaçable au rythme de l'aspiration, le couvercle (20) étant transparent au moins pour la plus grande partie et étant muni d'une fermeture rapide (17, 18), et la membrane étant sous forme d'une membrane mobile qui est serrée exclusivement au niveau de son bord externe (16.1).

2. Pompe à lait maternel selon la revendication 1, caractérisée en ce que la membrane de séparation (16) décrit une course qui est de l'ordre de grandeur de son diamètre.

3. Pompe à lait maternel selon la revendication 1 ou 2, caractérisée en ce que la membrane mobile (membrane de séparation 16) consiste en une matière plastique susceptible d'être désinfectée, de préférence à base de silicone, renforcée par un tissu.

4. Pompe à lait maternel selon au moins l'une des revendications précédentes, caractérisée en ce que la membrane de séparation (16) présente au bord un bourrelet de serrage et d'étanchéité (16.2).

5. Pompe à lait maternel selon au moins l'une des revendications précédentes, caractérisée en ce que le couvercle transparent (20) en tant que pièce en matière plastique unique consiste en une matière plastique transparente.

6. Pompe à lait maternel selon au moins l'une des revendications précédentes, caractérisée en ce qu'un filetage de vissage (bord de filetage interne 18) et des nervures de préhension (20.1) sont associés au couvercle (20).

7. Pompe à lait maternel selon au moins l'une des revendications précédentes, caractérisée en ce qu'une tubulure (92) de raccordement à un tuyau souple avec une ouverture d'air supplémentaire (96) réglable est disposée dans le couvercle (20), de préférence en position centrale.

8. Pompe à lait maternel selon au moins l'une des revendications précédentes, caractérisée en ce que la tubulure (92) de raccordement à un tuyau souple est vissée à bloc dans une ouverture (91) du couvercle (20) et porte un filetage (92.4) de diamètre plus petit pour un écrou de raccord et de réglage (98) qui limite une fente conique entre un domaine d'étanchéité (collerette de réglage 98.1) et un siège (95) de la tubulure (92) de raccordement à un tuyau souple, l'écrou de raccord et de réglage (98) étant soutenu sur un ressort de soupape de réglage (101).

9. Pompe à lait maternel selon au moins l'une des revendications précédentes, caractérisée en ce que le piston (13, 213) de la membrane de séparation (16) sous forme de membrane mobile est guidé dans un corps de cylindre (11, 211).

10. Pompe à lait maternel selon au moins l'une des revendications précédentes, caractérisée en ce que le piston (13) est soutenu sur un ressort de compression (14).

11. Pompe à lait maternel selon au moins l'une des revendications 1 à 9, caractérisée en ce que la piston (13, 213) est entraîné par un mécanisme à bielle et manivelle (214, 215).

12. Pompe à lait maternel selon au moins l'une des revendications 1 à 8, caractérisée en ce que le piston est sous forme d'un piston double (223, 219) avec une deuxième membrane mobile (216) du même type et avec une tige de piston (221) guidée à glissement dans le corps, et en ce que la tige de piston (221) est actionnable.

13. Pompe à lait maternel selon au moins l'une des revendications 1 à 10, caractérisée en ce que le piston (13) chargé par ressort qui soutient la membrane de séparation (16) qui est sous forme de membrane mobile est guidé de façon étanche dans un cylindre à air qui peut être raccordé de façon rythmée par l'intermédiaire d'une soupape à manoeuvre intermittente (52, 123) à un générateur de dépression et qui peut être séparé de celui-ci et relié à l'atmosphère.

14. Pompe à lait maternel selon la revendication 13, caractérisée en ce qu'il est prévu un raccord de liaison (46, 115.1, 116) pour une installation d'aspiration centrale d'un hôpital ou analogue.

15. Pompe à lait maternel selon la revendication 13, caractérisée en ce qu'il est prévu un éjecteur à gaz (117) pour la production d'une dépression à partir d'un système à air comprimé central (118.1, 119) ou d'un générateur d'air comprimé séparé (118.2, 120).

16. Pompe à lait maternel selon la revendication 13, caractérisée en ce qu'un générateur de dépression séparé (115.3, 121) est associé à la pompe à lait maternel (10).

17. Pompe à lait maternel (210) selon la revendication 13, caractérisée en ce que la soupape à manoeuvre intermittente est une soupape à trois voies et deux positions (123, 124) actionnée électroniquement au moyen d'un générateur de séquence de synchronisation (125).

18. Pompe à lait maternel (10) selon au moins l'une des revendications 1 à 10 et 13 à 16, caractérisée en ce que la soupape à manoeuvre intermittente est un système de soupapes automatiques à multivibrateur (52) qui commande avec la dépression propre, de façon automatique du point de vue pneumatique.

19. Pompe à lait maternel selon la revendication 18, caractérisée en ce que le système de soupapes pneumatiques à multivibrateur (52) comporte deux soupapes à disque (38, 57) à manoeuvre alternée, parmi lesquelles l'une fonctionne au moyen d'un poussoir (80) soutenu par l'intermédiaire de ressorts (86, 87) et déplacé par une liaison glissante (28) active seulement dans les positions extrêmes du piston (13), et l'autre est actionnée par des forces pneumatiques actives des deux côtés d'une membrane (62) de séparation des chambres de soupape et par un ressort (70).

20. Pompe à lait maternel selon la revendication 19, caractérisée en ce que dans le fond (24) du cylindre de la chambre du cylindre d'actionnement (12) il est prévu un alésage central (32) par lequel le poussoir (80) pénètre dans la chambre de soupape (37) dans laquelle il porte un disque de soupape (81) qui, depuis la fin de la course de sortie du piston (13) qui se produit sous l'effet du ressort (14) et lors de l'entrée de l'air atmosphérique jusqu'à peu de temps avant la fin de la course d'entrée du piston (13) qui se produit sous aspiration, est situé à distance d'un disque d'étanchéité (41) disposé en face de lui au fond de la chambre de soupape (37) et qui présente un évidement central (42) qui est relié au raccord d'aspiration (45) par l'intermédiaire de canaux d'aspiraton (43.1, 43.2).

21. Pompe à lait maternel selon la revendication 19 ou 20, caractérisée en ce que la soupape à ressort (57) du système de soupapes pneumatiques à multivibrateur (52) présente un joint annulaire (58) dont l'espace central libre (56) est relié à l'atmosphère par l'intermédiaire d'une conduite atmopshérique (55) et sur lequel peut être appliqué par le ressort (70) un élément de fermeture de soupape à disque (73) à étanchéité annulaire qui est fixé sur la membrane (62) de séparation des chambres de soupape qui subdivise la chambre de soupape de commande en une chambre de soupape à disque (59) et en une chambre de soupape à ressort (65), la chambre de soupape à disque (59) étant reliée à la chambre de cylindre d'actionnement (12) par une conduite (60, 61), et la chambre de soupape à ressort (65) étant reliée au raccord d'aspiration (45), et le ressort (70) et les surfaces actives des deux côtés de la membrane (62) de séparation des chambres de soupape coïncident mutuellement de telle façon que la liaison à l'atmopshère est interrompue tant qu'il règne une pression sensiblement identique des deux côtés de la membrane de séparation des chambres de soupape, tandis que lors de la fermeture de la soupape à poussoir (38) actionnée par le poussoir (80), la dépression devient totalement active dans la chambre de soupape à ressort (65) et soulève l'élément de fermeture de soupape à disque (73) du joint annulaire (58) contre la force du ressort (70) et ouvre la liaison de la chambre de cylindre d'actionnement (12) à l'atmosphère (55, 50, 51, 54).

22. Pompe à lait maternel selon au moins l'une des revendications 19 à 21, caractérisée en ce que la conduite atmosphérique (55) du système de soupapes pneumatiques à multivibrateur (52) conduit, dans un fond de corps de pompe (34) épaissi du corps de pompe (11) sous forme de cylindre, à une chambre annulaire (50) qui est entourée par un filtre annulaire (51) en feutre ou en produit alvéolaire et qui est recouverte par un capuchon de fermeture de corps (48).

23. Pompe à lait maternel selon au moins l'une des revendications précédentes, caractérisée en ce que la membrane (62) de séparation des chambres de soupape de la soupape à ressort (57) du système de soupapes pneumatiques à multivibrateur (52) est maintenue par une pièce de tête (47) fixée sur le fond (34) du corps de pompe et de diamètre sensiblement identique, le raccord d'aspiration (45) présentant dans la pièce de tête (47) une tubulure de raccord d'aspiration (46) décalée par rapport à l'axe (90) du corps (11) formant le cylindre, les deux soupapes sous forme de soupapes à disque (soupape à poussoir 38 et soupape à ressort 57) étant disposées de façon centrée, et le ressort (70) de la soupape à ressort (57) étant muni d'une vis de réglage et de soutien (68) accessible de l'extérieur.

## Claims

1. A breast milk pump (10) with a membrane arranged movably in a pump casing (11) between a driveable piston (13) and a lid (20) which has an air inlet and outlet borehole (97), which membrane is clamped as a separating membrane between an end face (15) of the pump casing and the lid which can be removed from the casing (11), together with the lid (20) forms the boundary of the suction chamber (22), and can be moved in the suction rhythm, the lid (20) being transparent in design, for the most part at least, and equipped with a quick closure (17, 18), and the membrane being constructed as a rolling membrane which is clamped exclusively at its outer edge (16.1).

2. A breast milk pump according to Claim 1, characterised in that the separating membrane (16) executes a lifting movement which is of the order of magnitude of its diameter.

3. A breast milk pump according to Claim 1 or 2, characterised in that the rolling membrane (separating membrane 16) consists of a disinfectable synthetic material which is reinforced with fabric and is preferably on a silicone basis.

4. A breast milk pump according to at least one of the preceding Claims, characterised in that the separating membrane (16) has a clamping and sealing bead (16.2) on its edge.

5. A breast milk pump according to at least one of the preceding Claims, characterised in that the transparent lid (20) as a one-piece synthetic material part consists of transparent synthetic material.

6. A breast milk pump according to at least one of the preceding Claims, characterised in that a screw-over thread (internal thread edge 18) and handgrips (20.1) are associated with the lid (20).

7. A breast milk pump according to at least one of the preceding Claims, characterised in that a tube connection piece (92) with adjustable supplementary air opening (96) is arranged in the lid (20), preferably at the centre.

8. A breast milk pump according to at least one of the preceding Claims, characterised in that the tube connection piece (92) is screwed tight in an opening (91) in the lid (20) and carries a thread (92.4), which is smaller in diameter, for a sleeve and adjustment nut (98) which forms the boundary of a conical slit between a sealing region (adjustment collar 98.1) and a seat (95) of the tube connection piece (92), the sleeve and adjustment nut (98) being supported on an adjustment valve spring (101).

9. A breast milk pump according to at least one of the preceding Claims, characterised in that the piston (13, 213) of the separating membrane (16) constructed as a rolling membrane is guided in a cylindrical casing (11, 211).

10. A breast milk pump according to at least one of the preceding Claims, characterised in that the piston (13) is supported on a compression spring (14).

11. A breast milk pump according to at least one of Claims 1 to 9, characterised in that the piston (13, 213) is driven by a crank drive (214, 215).

12. A breast milk pump according to at least one of Claims 1 to 8, characterised in that the piston is constructed as a twin piston (223, 219) with a second similar rolling membrane (216) and with a piston rod (221) which is slidably guided in the casing, and in that the piston rod (221) is driveable.

13. A breast milk pump according to at least one of Claims 1 to 10, characterised in that the springloaded piston (13), which supports the separating membrane (16) constructed as rolling membrane, is guided in a sealed manner in an air cylinder which, by means of an intermittently switching valve (52, 123), can be connected rhythmically to a low-pressure generator and can be disconnected from this and connected to the atmosphere.

14. A breast milk pump according to Claim 13, characterised in that an attaching connection (46, 115.1, 116) is provided for a central suction installation of a hospital or the like.

15. A breast milk pump according to Claim 13, characterised in that a gas-jet pump (117) is provided for generating low pressure from a central compressed-air system (118.1, 119) or separate air compressor (118.2, 120).

16. A breast milk pump according to Claim 13, characterised in that a separate low-pressure generator (115.3, 121) is assigned to the breast milk pump (10).

17. A breast milk pump (210) according to Claim 13, characterised in that the intermittently switching valve is a three-way two-position valve (123, 124) which is operated electronically with time cycle control (125).

18. A breast milk pump according to at least one of Claims 1 to 10 and 13 to 16, characterised in that the intermittently switching valve is a multivibrator pneumatic-valve system (52) which exerts pneumatic control automatically with its own low pressure.

19. A breast milk pump according to Claim 18, characterised in that the multivibrator pneumatic-valve system (52) has two reciprocally switching poppet valves (38, 57), one of which works by means of a tappet (80) supported via springs (86, 87) and moved with a pulling connection (28) only effective in the end positions of the piston (13) and the other is operated with air forces effective on both sides of a valve space separating membrane (62) and with a spring (70).

20. A breast milk pump according to Claim 19, characterised in that a central borehole (32) is provided in the cylinder base (24) of the driving cylinder space (12) through which borehole the tappet (80) extends into the valve chamber (37) in which it carries a valve disc (81) which, from the end of the piston's (13) outward stroke, which occurs under the action of the spring (14) and on the inflow of atmospheric air, until shortly before the end of the piston's (13) inward stroke, which occurs under suction, lies at a distance from a sealing disc (41) arranged opposite the valve disc on the base of the valve chamber (37), which sealing disc has a central recess (42) connected via suction channels (43.1, 43.2) with the suction connection (45).

21. A breast milk pump according to Claim 19 or 20, characterised in that the spring valve (57) of the multivibrator pneumatic-valve system (52) has an annular seal (58) whose free central space (56) is connected via an atmosphere pipeline (55) with the atmosphere and onto which an annularly sealing poppet-valve closing-element (73) can be pressed by the spring (70), which closing element is attached to the valve-space separating-membrane (62) which divides the control valve space into a poppet valve space (59) and a spring valve space (65), and the poppet valve space (59) is connected via a pipeline (60, 61) with the driving-cylinder space (12), the spring valve space (65) is connected with the suction connection (45) and the spring (70) and the active areas on both sides of the valve-space separating-membrane (62) are coordinated in such a way that the connection to the atmosphere is interrupted for as long as approximately the same pressure prevails on both sides of the valve-space separating-membrane, whilst, on closure of the tappet valve (38) operated by the tappet (80), the low pressure becomes fully effective in the spring-valve space (65) and, against the force of the spring (70), lifts the poppet-valve closing-element (73) from the annular seal (58) and opens the connection of the driving-cylinder space (12) with the atmosphere (55, 50, 51, 54).

22. A breast milk pump according to at least one of Claims 19 to 21, characterised in that the atmosphere pipeline (55) of the multivibrator pneumatic-valve system (52) leads, in a thickened pump casing base (34) of the pump casing (11) constructed as cylinder, to an annular space (50) which is surrounded by an annular filter (51) made of felt or foam and covered by a casing cap (48).

23. A breast milk pump according to at least one of the preceding Claims, characterised in that the valve-space separating-membrane (62) of the spring valve (57) of the multivibrator pneumatic-valve system (52) is held by a head piece (47) which is mounted on the pump casing base (34) and has substantially the same diameter, and, in the head piece (47), the suction connection (45) is formed from a suction connection piece (46) which is offset in relation to the axis (90) of the casing (11) which forms the cylinder, both the valves constructed as poppet valves (tappet valve 38 and spring valve 57) are arranged centrally, and the spring (70) of the spring valve (57) is provided with an adjusting and supporting screw (68) which is accessible from the outside.

16

EP 0 123 269 B1

FIG. 1

FIG. 3

FIG. 2

FIG. 4

FIG. 6

FIG. 7